# EUROPEAN PATENT APPLICATION

(11) **EP 1 818 399 A1**
(43) Date of publication of application: **15.08.2007**
(21) Application number: 07250245.3
(22) Date of filing: 22.01.2007
(51) Int. Cl.: C12N 15/00, C12N 5/00

(54) **Methods for delivering extracellular target into cells**

(30) Priority: 23.01.2006 US 337109
(71) Applicant: National Yang-Ming University, Taipei 112 (TW)
(72) Inventor: Lin, Chi-Hung, Taipei 106 (TW); Lian, Wei-Nan, Taipei 105 (TW)
(74) Representative: Wright, Simon Mark

(57) **Abstract**

The invention relates to a method for intracellular delivery of substances, comprising the steps of suspending the substance in aqueous solution and providing sufficient speed to enable the suspended substance to penetrate the cell surface and become incorporated into the cell. This method does not require the accompaniment or aid of any particle, and both the substance and the cell retain their biological activity after the entry of substance into the cell.

## Description

### FIELD OF THE INVENTION

This invention relates to methods for delivery of a target (eg. substance) into one or more cells.

### DESCRIPTION OF PRIOR ART

Delivery of bioactive molecules to intact living cells or tissues can be achieved by a variety of chemical or physical methods. Some of the commonly used fluorescence dyes, such as Hoechst 33258 and Lucifer Yellow, cannot directly cross the plasma membrane by themselves; these probes are typically microinjected, or "permeabilized" into cells that are lightly extracted by mild detergent treatments (Arndt-Jovin and Jovin, 1989, In "Methods in Cell Biology", Ch. 16, pp. 417-448, Academic Press, New York; Swanson et al., 1987, J. Cell. Biol., 104: 1217-1222).

Introduction of bioactive molecules into cells can also be achieved by chemical means. In the conventional "transfection" procedure, DNA molecules can be effectively delivered to cultured cells by using calcium phosphate precipitation, or by coating with cationic lipid or being included in the liposomes (Bergan et al., 2000, Pharm. Res., 17(8): 967-973 ; Hsiung et al., 1982, Mol. Cell. Biol. 2(4): 401-411); however, such a process has not been successfully applied to the delivery of (exogenously made) recombinant proteins. Moreover, certain types of cells (such as neuronal cells or functionally well-differentiated cells) have been shown to be more resistant to such transfection protocols than others. In fact, gene deliveries to primary cultured neurons and/or polarized epithelial cells (such as MDCK and Hep G2 cells) are notoriously difficult.

DNA delivery can also be accomplished by biological means. Recent progress has made packing and delivering biomolecules into living cells possible both in *vitro* and *in vivo* by adenovirus, retrovirus, and papillomavirus (Fujita et al., 1995, J. Virol., 69(10): 6180-6190; Kalpana, 1999, Semin. Liver Dis., 19(1): 27-37; Sverdrup et al., 1999, Gene Therapy, 6: 1317-1321); again, such protocols do not allow delivery of recombinant proteins.

In another approach, bioactive molecules (such as DNA or proteins) can be linked or physically absorbed onto a solid substrate carrier (such as microparticles). The microparticle carriers can then be accelerated to a very high speed in the medium such that they can penetrate through the plasma membrane without significantly damaging the cell, as has been described in the so-called "gene gun" method (Sanford et al., 1987, Partic. Sci. Technol., 5: 27-37). Although this method possesses the advantage in penetration depth, especially when it is used in plant cells/tissues or animal skins, the presence of the delivered particles within the cytosol may unequivocally cause some adverse effects of the resident cell. Note also that the coating and absorption of bioactive molecules on the delivered particles by conventional gene gun protocols are better developed for DNA than proteins.

However, using gene gun techniques for delivering DNA into cells still has some problems. The gene gun is a particle delivery techniques where, the "bullet" is essential. DNA has to be coated on to a microparticle to penetrate the cell membrane. The microparticle commonly used is a gold particle or tungsten particle. Preparation of the bullet includes pre-treating the dried powder of microparticle by glycerol, coating the DNA on the pre-treated microparticle, spreading the coated microparticle on to a plastic tube, and cutting the plastic tube into pieces to act as the bullet. The process has disadvantages such as including complicated steps, it is time-consuming and has a high technology threshold. Further, the pre-treated microparticle has a limited preservation period and using gold and tungsten particles gives high costs.

The methods of present invention seeks to overcome, or at least mitigate, some of these disadvantages and provide an easier and or more effective process.

### BRIEF DESCRIPTION OF THE DRAWINGS

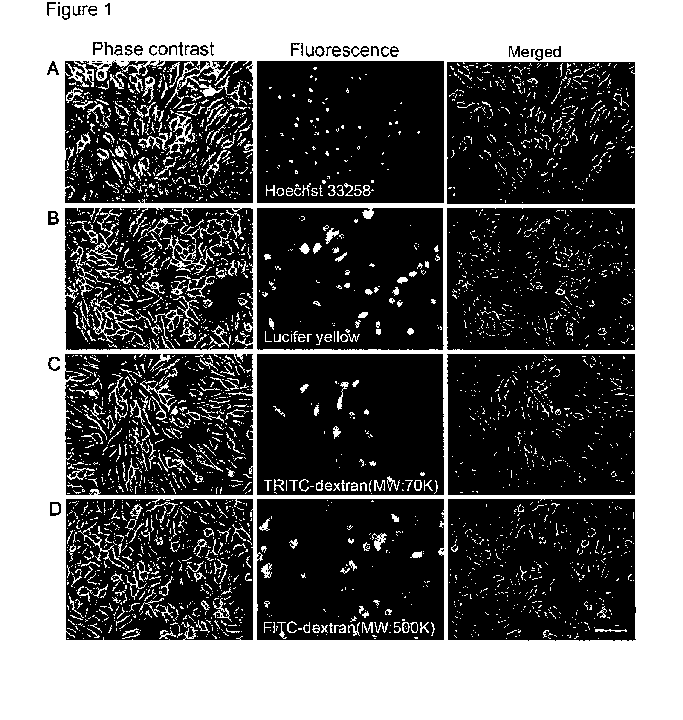

Figure 1 illustrates the delivery effects of chemical molecules of different sizes and properties. Cell morphology is observed under phase contrast microscope, and fluorescence signals are observed under fluorescence microscope. CHO cells delivered with Hoechst 33258 are shown in (A), the result is detected immediately after PBS wash. Signals of Lucifer yellow are shown in (B). Results of delivered dextrans (MW:70K) conjugated with TRITC and dextrans (MW:500K) conjugated with FITC are shown in (C) and (D). Scale bar indicates 20 µm.

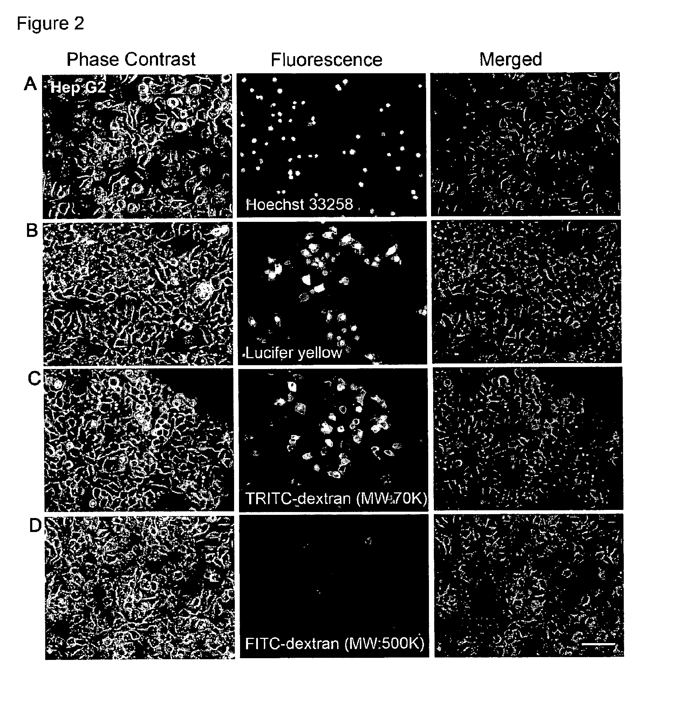

Figure 2 illustrates the delivery of chemical molecules in different cell types. Hep G2 cells are used in chemical molecules delivery assay. (A) Hoechst 33258, (B) Lucifer yellow, (C) dextrans (MW:70K) conjugated with TRITC and (D) dextrans (MW:500K) conjugated with FITC are observed under phase contrast and fluorescence microscopy. Scale bar indicates 20 µm.

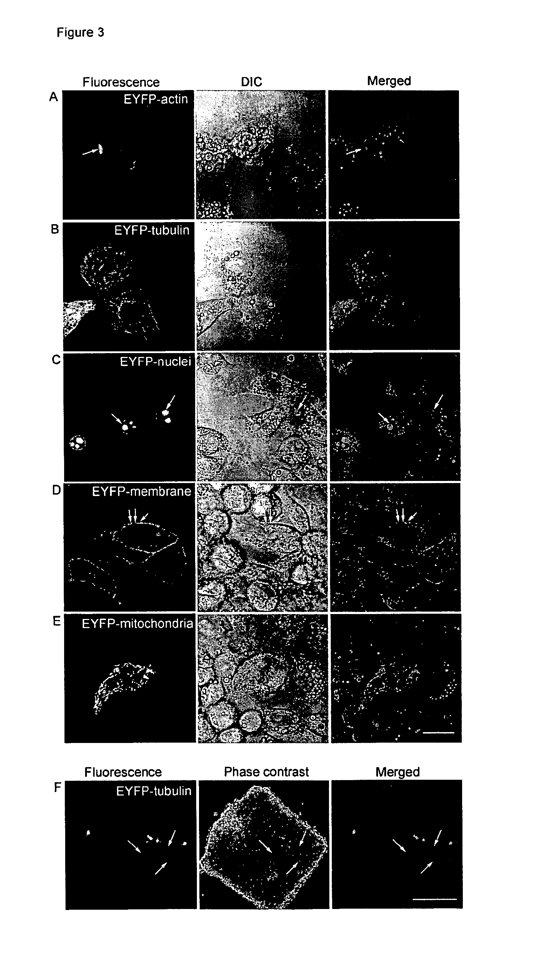

Figure 3 illustrates the delivery and expression of plasmid DNA in different cell types. EYFP fused subcellular localization plasmids are used for delivery and expression; the results are recorded by confocal microscopy with fluorescence and DIC images. (A) Expressed EYFP-actin shows submembrane pattern and the apical domain can be found *(arrow).* (B) Filamentous structure can be easily observed in Hep G2 cells with expression of delivered EYFP-tubulin plasmids. (C) Expression of EYFP-nuclei shows nuclear morphology and condensed signal in nucleolus *(arrows).* The nuclear identification can be also found in DIC images. (D) Membrane localization by expressing EYFP-membrane plasmid can be found in delivered Hep G2 cells *(arrows).* (E) Expression of EYFP-mitochondria plasmids reveals mitochondria localization in Hep G2 cells. Scale bar indicates 10 um in (A) ~ (E). (F) EYFP-tubulin plasmids are delivered into retina explants of goldfish and observed under microscope after 24hr. Neuronal axon which expressed EYFP-tubulin can be found under fluorescence microscopy *(arrows).* Scale bar indicates 200 µm.

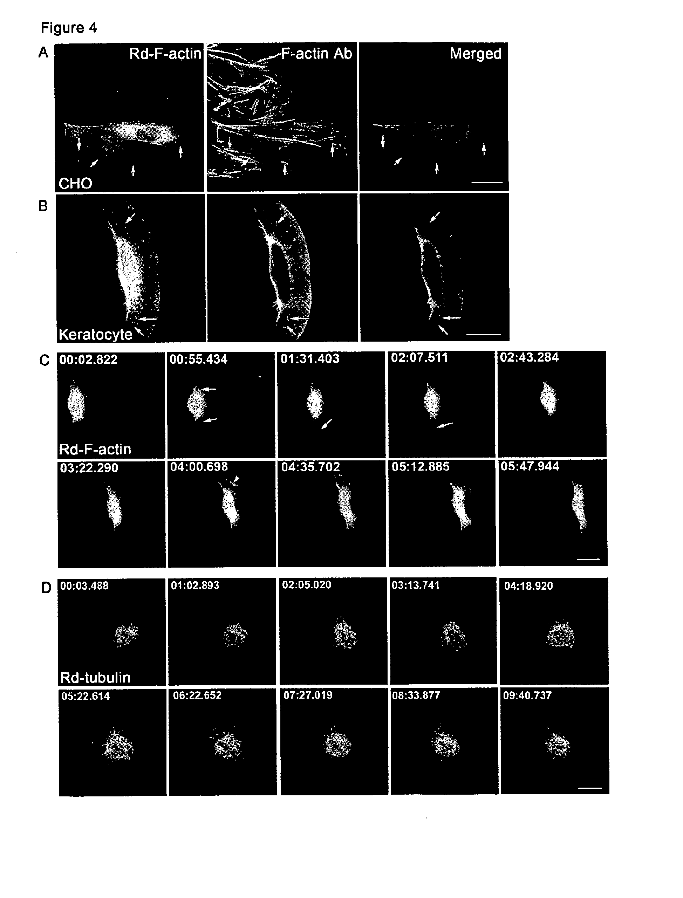

Figure 4 shows the delivery of fluorescence labeled actin and tubulin monomers into cells. Rhodamine (Rh)- labeled actin and tubulin monomers are used. (A) CHO cells are fixed and stained with Rd-Ph. Rh-labled monomeric actin assembles into actin filaments (*arrows*). (B) Fish keratocytes are subjected to the monomer delivery and observed under fluorescence microscope. Fixed and stained with actin antibodies shows the assembled actin filaments after incubation and colocalization in several stress fibers can be easily found *(arrows).* (C) Live fish keratocytes delivered with Rd-F-actin are observed under fluorescence microscope and recorded with time-lapsed imaging system. (D) The same experimental setup is performed in Rd-F-tubulin delivery in fish keratocyte. Scale bar indicates 10 µm.

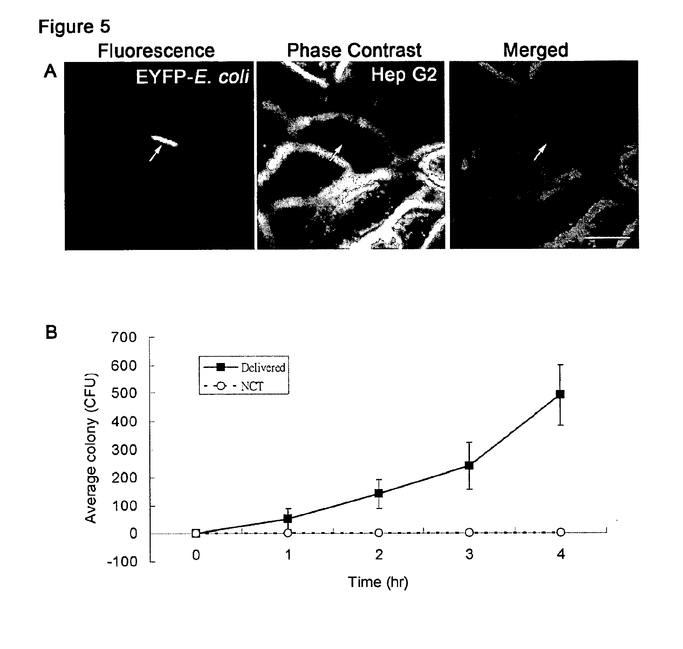

Figure 5 shows the delivery and replication of E. coli in cells. EYFP-*E*.*coli* is delivered into Hep G2 cells and gentamycin is supplement with cell culture medium for further incubation. (A) After delivery, Hep G2 cells are treated with gentamycin for 1hr and observed under fluorescence and phase contrast microscope. E. coli inside of cells are found *(arrow).* (B) Cells treated with gentamycin for 1hr and lysed for colony formation assay are determined as time point 0. At a different time point, gentamycin is supplemented with cell culture medium until cell lysed to inhibit the bacteria growth. The growth curve of delivered *E.coli* is recorded (*filled square*). Negative control is performed with the same amount of *E.coli* and put into cell culture medium with Hep G2 cells with gentamycin *(open circle).* The results were collected from four independent experiments. Mean ± SD is shown.

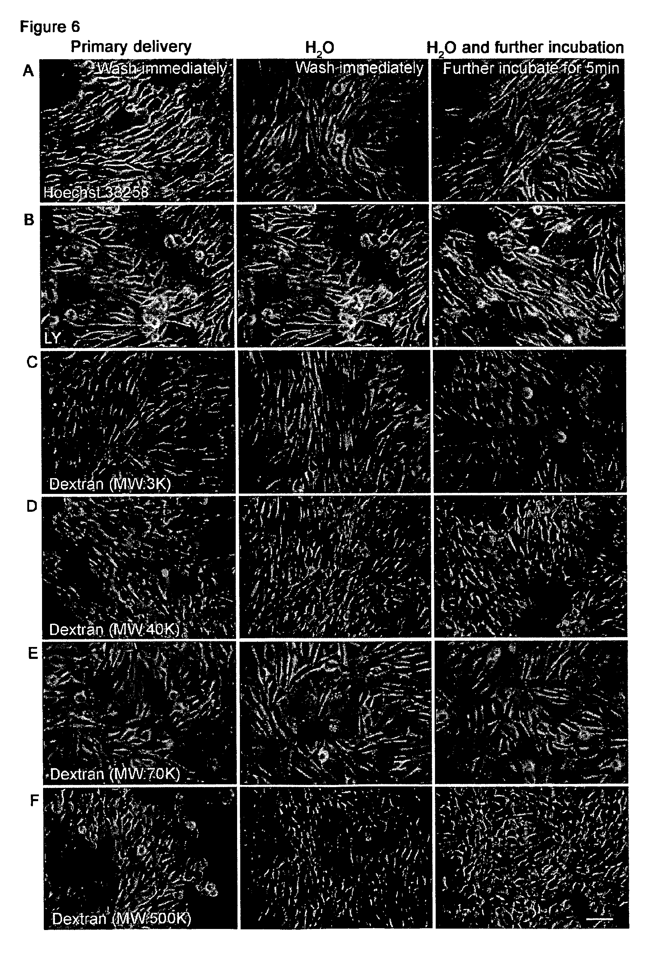

Figure 6 shows direct and indirect mechanisms to deliver molecules. Different molecules are delivered into CHO cells. In a direct delivery assay, fluorescence probes are put on the membrane for direct delivery. Cells are washed immediately with PBS *(left).* In indirect delivery assays, the same volume of the H₂O is put on the membrane to replaced fluorescence probes. Probes are mixed with cultured medium with CHO cells. After being shocked, cells are washed immediately *(middle)* or further incubated for 5 min and washed twice with PBS *(right).* Cells are observed under phase contrast and fluorescence microscope. (A) Hoechst 33258, (B) lucifer yellow, (C) TRITC labeled (MW:3K), (D) (MW:40K), (E) (MW:70K) dextrans, and (F) FITC labeled dextrans (MW:500K).

### SUMMARY OF THE INVENTION

This invention provides a (eg. *in vitro)* method for delivering a target into cells comprising: (a) optionally, mixing the target with a liquid (eg. solution) to form a mixture, and (b) delivering the mixture (into a solution containing) the cells by high pressure force (or using a fluid at high pressure). The invention is the delivery of the target into a cell (eg. by penetration or transformation) using a high pressure force technique, such that the target penetrates or is lodged inside the cells (or at least crosses, or passes through (an external) cell membrane).

This invention also provides a cell used for or in the method described as above, which facilitates the absorption of exogenous target into the cell based on the condition of the membrane of the cells by high pressure force. The high pressure (force) can be (applied or transmitted) by a fluid, such as a liquid (eg. water) or gas (eg. He). The target may not be present, attached to or coated on particle(s). The target may be accelerated by a gas (eg. He) at high pressure.

This invention further provides a (eg. *in vitro)* method for indirectly delivering a target into (one or more) cells, comprising: (a) mixing the target and the cells, and (b) changing the condition of the membranes of the cells (eg. increasing permeability) by high pressure force (or using a fluid at high pressure).

Preferred features of the aspect of the invention are applicable to another aspect *mutatis mutandis.*

### DETAILED DESCRIPTION OF THE INVENTION

"Target" used in this invention means a substance or composition to be, or is, delivered into cells.

Macromolecule: A (very) large molecule, such as a polymer or protein, usually consisting of many smaller structural units (eg. monomers) linked together.

This invention provides a (eg. non-particular) method for delivering a target into cells comprising: (a) mixing the target with a solution (or liquid) to form a mixture, and (b) delivering the mixture (into a solution containing) the cells by high pressure force. The method herein can be characterized as non-particle delivery (eg. particles are not used or can be avoided). Thus the target may not be carried or attached to on particles.

The target delivered into cells in this invention is selected from the group consisting of chemical, fluorescent compound, molecule exhibiting bio-activity, micromolecule, macromolecule or microorganism. After the delivery, the target may still maintain its function or activity in the cell.

In one embodiment, the chemical is dye, fluorescent dye, polysaccharide, or pharmaceutical compound. In one embodiment, the molecule exhibiting bio-activity is enzyme, drug, vaccine, antigen or antibody. In one embodiment, the macromolecule is protein, polypeptide, RNA, DNA, or other oligonucleotide. Further, the oligonucleotide can be a promoter, enhancer, siRNA, morpholino or other regulatory sequence.

The target can also be (or comprise) a cell usually of a smaller size than the cell to which it is delivered. In one embodiment, the target (or cell) comprises a microorganism. In a preferred embodiment, the microorganism is bacterium or yeast. In a more preferred embodiment, the microorganism is transformed bacteria or yeast. Further, the microorganism may maintain its life cycle and propagation capability (it may be viable) in the cell after delivery.

The cell delivered with an (exogenous) target herein can be a prokaryotic cell or eukaryotic cell. The cell can be selected from the group consisting of animal, insect, plant, fungus, alga, bacterium, rickettsia or *Chlamydia.*

The solution used in this method can be charged or uncharged. In a preferred embodiment, the solution is water, phosphate saline buffer or other media.

The high pressure force can be produced by a gene gun. The gene gun can be available from commercial producer. The conditions of gene gun use in this method can be set according to the experimental situation such as cell type, target type or other factors.

The method of the invention can be applied in various fields, for example but not limited, the field of gene therapy and the field of evolutionary study.

The present invention also provides a cell used for the method described as above, which facilitates the absorption of exogenous target into the cell based on the condition of the membrane of the cells by high pressure force. The target maybe a dye, fluorescent dye, polysaccharide, pharmaceutical compound, fluorescent compound, enzyme, drug, vaccine, antigen, antibody, protein, polypeptide, RNA, DNA, promoter, enhancer, siRNA, morpholino, regulatory sequences or a microorganism.

The invention further provides a method for indirectly delivering a target into cells, comprising: (a) mixing the target and the cells, and (b) changing the condition of a membrane of the cells by high pressure force. This method can be characterized as non-particle delivery. More detailed, high pressure force can be used in this method to alter the strength of cell membrane and to loosen the membrane. The (pressure) force may also cause the mixture containing cells and targets to shake to increase the opportunities of collision between cells and targets. Based on the above , the target may be able to pass through the cell membrane and be absorbed by cells.

In a more preferred embodiment, the high pressure force is produced by gene gun.
The target can be a dye, fluorescent dye, polysaccharide, pharmaceutical compound, fluorescent compound, enzyme, drug, vaccine, antigen, antibody, protein, polypeptide, RNA, DNA, promoter, enhancer, siRNA, morpholino, other regulatory sequences, bacterium or yeast. The cell is selected from the group consisting of animal, insect, plant, fungus, bacterium, rickettsia and *Chlamydia.*

The method of the invention can be applied in various fields, for example but not limitation, the field of gene therapy and the field of evolutionary study.

The methods provided by this invention also can be applied in producing transgenic plant or transgenic animal. The transgenic plant may include corn, rice, soybean, potato, rape, cotton, mushroom, orchid, crops and garden plants. The transgenic animal may include pets, domestic animals, domestic fowl, experimental animals or (even) a protein-producing animal.

The methods of this invention can be applied in gene therapy, for example but not limited to, DNA vaccine or cancer therapy. Further, the methods of this invention can deliver not only DNA but also chemical and bioactive compound(s). This may enlarge the field of gene therapy.

For the evolutionary study, it is believed that extreme conditions such as an unstable atmosphere, continued thunder and lightening, volcanic eruptions, and earthquakes occurred in the youth of the Earth. The first life form is speculated to appear on Earth in this age. The primitive cell that first appeared in the world is speculated to have absorbed other molecules such as RNA, perhaps actively or passively, during evolution. The force for absorbing is speculated to be similar to an extreme environment, such as providing thunder and lightning. This invention provides a method of subjecting a cell to a high pressure force. The method can be used to mimic the conditions described as above and can be applied to study biological evolution.

### EXAMPLE

### Materials and Methods

### Cell lines

CHO (Chinese Hamster Ovary) and Hep G2 cell lines were used in this investigation. Standard cell culture protocols were followed. Cells were typically cultured with DMEM (GIBCO BRL) supplemented with 10% fetal calf serum, 2mM L-glutamine, and 5% non-essential amino acids (GIBCO.BRL) and incubated at 37°C in the presence of 5% CO2.

### Primary cells

For isolation of retinal explants and keratocytes, goldfish were anesthetized with 0.05% ethyl 3-aminobenzoate (Sigma). Optic nerve crushes were administered by crushing the exposed nerve behind the orbit. Nerve crushed fish were stored in water tank for 7 days in room temperature. Before retina isolation, the fish was dark-adapted for 30 min and then anesthetized. Eyes were removed and put into the Hanks' buffer (Sigma). The retina was isolated from eyecup, and chopped into 0.4 mm square pieces. Isolated explants were washed with Hanks' buffer. Retina explants were plated onto poly-L-lysine coated coverslips for plasmid delivery.

Fish keratocytes were isolated from fish scales. In brief, fish scales were extracted with tweezers, and placed on dry coverslips until almost dry. Hepes-DMEM (GIBCO Life Technology) supplemented with 10% fetal bovine serum, and 0.1% gentamicin solution was used to culture over night and allow the keratocyte migrate out of the fish scales. Migrated keratocyte were trypsinized by Trypsin EDTA. Collected keratocytes were placed on the sterilized coverslips for molecule delivery.

### Chemicals, polysaccharides, plasmids and proteins

All fluorescence marker and fluorescence labeled dextrans were purchased from Molecular probes (Molecular probes, Eugene, Oregon). For each shot, Hoechst 33258 was used in 3µg, lucifer yellow was used in 15µg. TRITC labelled dextrans (MW:70K) were used in 60µg, and FITC labelled dextrans (MW:500K) were used in 60µg. EYFP fused subcellular localization plasmids were purchased from Clontech. Plasmids were used in 6µg for each experiment. Rhodamine conjugated actin and tubulin (Cytoskeleton, Denver) were used in 40µg every shot.

### Transformation

Competent cells (strain BL21-DE) for bacteria transformation were prepared by calcium chloride. The optimal optical density (OD600) range for competent cell preparation was 0.15-0.45. Conventional transformation was performed. In brief, 50 µl competent cells with 1µg/µl plasmid DNA (pTriEx-3 Vector from Novagen) were mixed, incubated for 30 min at 37°C, heat shocked at 42 °C for 90 s, transfered to ice for 2 min, added with 100 µl liquid LB medium, and then recovered at 37°C for 45 min. Transformed bacteria was spread on the LB plate with ampicillin at 37°C overnight.

### Colony formation assay

Hep G2 cells were cultured on 10cm dishes for 24 hrs for bacteria delivery assay. Cells were washed with PBS twice immediately after bacteria delivery, and divided into five 35mm dishes by trypsinization. Cell culture medium with gentamycin (100µg per ml) supplement was used to inhibit extracellular bacteria activity. In the period of time indicated in experiments, cells were lysed with NET buffer (150mM NaCl, 0.5% NP-40, 50mM Tris, 1mM EDTA and 1% Triton X-100, supplemented with protease inhibitors) for 30 min at 4°C. Cell pellets were collected after 12,000xg centrifugation at 4°C for 30 min, and spread on the LB plates with ampicillin (40 µg/ml). Cultured overnight at 37 °C incubator and formed colony were first confirmed by immunofluorescence microscope and counted for statistics. Colony formation number was averaged from 4 independent experiments and standard deviation was provided.

### Solution accelerated method

The target for delivery was mixed with a solution and accelerated by high-pressured pure helium gas. PDS-1000/He system (Bio-Rad) was used to supply the high velocity. The bombarded volume was 6 µl in every experiment. The target cells were put on the target shelf and the compositions for delivery were loaded on the microcarrier membrane. Then, the rupture disk was assembled to the rupture disk retaining cap and the microcarrier was assembled on the macrocarrier cover as described in the user guide (Bio-Rad). The door of the bombardment chamber was closed; the suction was turned on to vacuum the chamber. When the degree of the vacuity was sufficient, the helium tank was turned on to supply the high velocity. The high pressure forced the target penetrating the cells. After every shot, the cells were immediately washed with PBS twice and filled with fresh medium to avoid biological endocytosis. Cells were then observed under fluorescence microscope (Leica GmbH, Heidelberg, Germany) or further incubated if need.

### Imaging study

Every sample was observed immediately after bombardment, except for plasmid delivery and rhodamine conjugated actins and tubulins. Plasmid delivered cells were further incubated for extra 48hrs at 37°C. Fluorescece labeled proteins were incubated at 37°C for 30min after delivering. All images were taken by SIT camera (Hamammatsu 2400) and analyzed by Metamorph (Universal Imaging Corporation, West Chester, PA), or by confocal microscope (Leica GmbH, Heidelberg, Germany).

### Statistics

Cells with positive signals under microscope were calculated field by field and versus total cell number. Finally, total cell number was normalized into 1000 in terms of comparison.

### Immunostaining

For immunofluorescence staining, cells were typically cultured on a 22 x 22 mm square coverslip which was pretreated with 6N HCl and 95% ethanol, and coated with 200 g/ml poly-L-lysine (MW 70-150 KDa, Sigma) as previously described (Lian et al., 1999, Hepatology, 30(3): 748-760; Lin and Forscher, 1993, J. Cell Biol., 121(6): 1369-1383). Cells were fixed with 4% paraformaldehyde/2 mM EGTA/400 mM sucrose/PBS at RT for 15 min, then permeabilized with 0.5% Triton X-100 in the fix solution for 5 min. The samples were then incubated with 5 mg/ml BSA/PBS, then with primary antibodies at RT for 1hr. The concentrations of primary antibodies utilized were 1:100 anti-beta-tubulin antibody (Sigma). After extensive PBS washes, fluorophore-conjugated secondary antibodies (Jackson Immuno Research, West Grove, PA) were added at the concentrations recommended by the manufacturer at room temperature for 1 hr. About 1 unit/ml FITC-Ph was used for F-actin staining. The stained samples were mounted using an anti-photobleaching medium containing 20 mM n-propyl-gallate (Sigma) in 80% glycerol/20% PBS All images were recorded in a digital platform for data analysis and image processing.

### EXAMPLE 1: Delivery of fluorescent compounds and polysaccharides in different sizes into different cell-types

To demonstrate the molecules delivery through solution accelerating methods, CHO (Chinese Hamster Ovary) cells were first used. The bisbenzimide dye-Hoechst 33258 is a fluorescence chemical compound, which can bind to the minor groove of double-helix DNA. The molecular weight of Hoechst is 623.96. Hoechst 33258 is used as a nucleic marker due to its fluorescence characteristic and high affinity to DNA. Usually, Hoechst 33258 can only be used in fixed cells or detergent-permeable cells, although, Hoechst 33258 has been reported with slightly lower permeability efficiency than Hoechst 33342 (Arndt-Jovin and Jovin, 1989, In "Methods in Cell Biology", Ch. 16, pp. 417-448, Academic Press, New York). Invisible signals were found while cells were incubated with Hoechst 33258 for 5 minutes. The solution accelerating method provided an extremely short period of time for contact between molecules and the cell membrane.

To discriminate the results from endocytosis, cells were washed twice with PBS and changed with new medium for further incubation every shot. Compared with results from incubating cells with soluble molecules, the solution accelerating method performed a quick and harmful-less delivery route.

The result of Hoechst 33258 was shown in Figure 1A. The concentration of Hoechst 33258 was diluted into 1/10. Delivery rate was reduced after dilution (Table 1). Hence, it was found that molecule using solution accelerating to deliver showed a dose dependent manner.

*N*-(2-aminoethyl)-4-amino-3,6- disulfo-1,8-naphthalimide, dipotassium salt (lucifer yellow ethylenediamine) has the molecular weight in 491.57 (Stewart, 1981, Nature, 292 (2): 17-21). Lucifer yellow CH (LY-CH or LY) was used to determine whether the polar trace molecule can be delivered through this unique solution accelerating method used in the present invention. LY is an impermeable fluorescence dye, which is often used as a microinjection marker and can not be transported through gap junction (Powley and Berthoud, 1991, J. Neuro. Methods, 36: 9-15). LY showed the similar results, which can be delivered through solution accelerating method and showed the dose dependent manner (See Table 1). In Figure 1B, fluorescence signal from LY was found in entire cell.

Dextran is a hydrophilic polysaccharide synthesized by *Leuconostoc* bacteria. Dextrans have been widely used in various biological applications due to their high water solubility and low toxicity. Dextrans are available in different sizes and different fluorochrome-labeled forms. Dextran (MW:70K) is used as an endocytosis marker (Makarow, 1985, EMBO J., 4(7): 1861-1866). Here both TRITC labeled dextran (MW:70K) and FITC labeled dextrans (MW:500K) were used to demonstrate delivery of different molecule sizes. It was found that dextran (MW:70K) can be transferred into cells by the method of the invention. To avoid the possibility that the cytosolic signal may come from endocytosis, soluble dextrans (MW:70K) were incubated in the same concentration for 5 minutes in 37°C. No significant signals in cells were found.

Therefore, large molecule delivery of dextran (MW:500K) was further tested. In Figure 1C and D, both dextrans (MW:70K) and dextrans (MW:500K) were successfully introduced into cells as small molecules, demonstrating that the method of the present invention can deliver not only small molecules but also large molecules in liquid phase. Both dextrans (MW:70K) and dextrans (MW:500K) showed dose dependent manner (Table 1). Interestingly, the dose-dependent effect was more obvious in dextrans (MW:500K) (reduced from 39.1 to 8.28% while the concentration is half) than in dextrans (MW:70K) (reduced from 38.16 to 25.44% while the concentration id half) dextrans. The result supported that the method of the present invention may play more than one role in molecules delivery, or this method is restricted in large molecules with lower concentration.

**Table 1. Molecule delivery efficiency by the solution accelerating method**

| Target cells | Molecules delivered | | Concentrations* | Delivery efficiency# (targeted cells/500 cells) |
|---|---|---|---|---|
| | Hoechst | 33258 | 1 mg/ml | 65.48±18.81 |
| | (M.W. = 623.96) | | 0.5 mg/ml | 57.7±25.56 |
| | | | 0.1 mg/ml | 27.25±7.22 |
| | Lucifer | yellow | 2.5 mg/ml | 35.47±8 |
| | (M.W. = 491.57) | | 1.25 mg/ml | 19.9±2.64 |
| CHO | | | 0.5 mg/ml | 11.95±3.36 |
| | | | 10 mg/ml | 38.16±8.19 |
| | 70kD dextran | | 5 mg/ml | 25.44±9.46 |
| | | | 2.5mg/ml | 19.04±4.61 |
| | | | 10mg/ml | 39.1±8.79 |
| | 500kD dextran | | 5mg/ml | 8.28±2.19 |
| | | | 2.5mg/ml | 4.35±2.66 |
| CHO | DNA (GFP-tubulin gene) | | 1 mg/ml | 34.82±5.07 |
| Hep G2 | DNA (GFP-tubulin gene) | | 1 mg/ml | 9.06±1.85 |
| CHO | Actin proteins | | 3 mg/ml | 67.15±13.04 |
| CHO | Tubulin proteins | | 3 mg/ml | 62.36±5.89 |

| | | | | |
|---|---|---|---|---|
| * Concentrations of the solutions used for delivery are shown; delivery volume = 6 ml. # Mean +/- STDEV is shown. | | | | |

To rule out the possibility that using solution accelerating method may be discriminated by distinct cell models, the same experiments were carried out in human liver blastoma cell line, Hep G2 cells, to see whether the delivery could be achieved or not. In Figure 2A-B, Hoechst 33258 and LY were successfully delivered into Hep G2 cells by solution accelerating method. The delivered efficiency was not affected by different cell types from statistic results in CHO cells (Table 1) and Hep G2 cells (Table 2, probe direct bombardment). Both dextran (MW:70K) and dextran (MW:500K) were delivered to Hep G2 cells (Figure 2C-D). Efficiency comparison can be found from Table 1 and Table 2, showing that the molecules delivery through solution accelerating method is not cell type specific manner.

### EXAMPLE 2: Delivery and expression of plasmid DNA in different cell types

A particle carrier method (gene gun) has been widely used for DNA delivery (Johnston and Tang, 1994, in "Methods in Cell Biology", vol. 43, ch. 17, pp. 353-365, Academic Press). Similar to the method with high-pressured helium which accelerates plasmid DNA, accelerating soluble plasmid DNA (EYFP fused subcellular localization plasmids) alone without any particle carriers was tested herein. After delivery, Hep G2 cells were further incubated for 24 hr and observed under confocal microscope. Interestingly, accelerated soluble plasmids were delivered and expressed in cells without particle carriers. Hep G2 cells are polarized hepatocytes. Membranes in polarized Hep G2 cells were distinguishable from basolateral domain and apical domain. Apical domain in Hep G2 cells was full of microvilli and actin accumulated signals were detectable by F-actin staining (Lian et al., 1999, Hepatology, 30(3): 748-760).

Expression of EYFP-actin by solution accelerating methods was found to be located on both submembrane and apical domain (Figure 3A, *arrows)* in Hep G2 cells. EYFP signals in microtubule filaments can be found in Hep G2 (Figure 3B).

EYFP-nuclei showed nuclear localization, strong EYFP signal was observed in nucleolus (Figure 3C, *arrows*)*.* Nuclear and nucleolus morphology were confirmed by DIC images (Figure 3C). EYFP signal can be found on cell membrane by the expression of EYFP-membrane plasmid (Figure 3D, *arrows*). The expression of EYFP-mitochondria represented the mitochondria localization in Hep G2 cells (Figure 3E). These results indicated that without particles as carriers, plasmid can be delivered and express in cells properly. Different cell line were also used for plasmid delivery experiments (Table 1), different cell type affected the delivery efficiency.

Tissue explants can be difficult to deliver with a plasmid for expression. Goldfish retina explants were used to test whether the plasmid delivery can be used by solution accelerating method or not. It was shown that EYFP signal can be observed in neuronal cells inside of retina explant (Figure 3F, *arrows).* This result demonstrated that DNA delivery without particle carriers can be performed both on cells and tissues.

### EXAMPLE 3: Delivery of proteins and maintenance of its activity in different cell types

Delivery of proteins can also be difficult to achieve in cellular level, especially for large peptides. In previous studies, researchers have tried different methods to accomplish this issue, such as using a short peptide vector which contains a hydrophobic region as a carrier (Hawiger, 1997, Curr. Opin. Immunol., 9:189-194; Morris et al., 1997, Nucle. Acids Res., 25(4): 2730-2736), or incorporating a palmitoyl-lysine residue into the N- or C-terminal end to deliver the short hydrophilic peptides (Loing et al., 1996, Peptide Research, 9(5): 229-232). Moreover, Johnson et. al., have used saponin to produce the transient cell membrane permeabilization in neonatal cardiac myocytes, which showed they successfully introduced 125 I-labeled calmodulin and a 20 KDa protein kinase C epsilon fragment into the cells (Johnson et al., 1996, Circulation Research, 79: 1086-1099). Recently, Pep-1, a cell penetrated peptide was reported from Morris et. al, (Morris et al., 2001, Nature Biotechnology, 19: 1173-1176) . It showed a powerful delivery function in varies of peptides and proteins.

In the present invention, whether proteins can be delivered into cells through soluble acceleration method or not was also examined. Rhodamine labeled actin and tubulin were tested in solution phase. After proteins were delivered, cells were washed with PBS twice immediately to avoid further uptake the soluble exogenous proteins in medium. After washing out the non-delivered proteins, cells were further incubated for 30 minutes in cell culture incubator to examine whether exogenous proteins can be assembled or not. CHO cells and fish keratocytes were used in protein delivery experiments. In order to confirm the F-actin or microtubule filaments, immuno-fluorescence was also performed by FITC-phalloidin and anti-tubulin antibodies. Distribution pattern from rhodamine and FITC showed almost co-localization, which represented that delivered rhodamine-actin can not only entry cell membrane successfully but also incorporated into actin assembly (Figure 4A-B, *arrows).* The increasing cytosolic rhodamine signals in delivered cells were also found, probably due to the free form rhodamine labeled actin. Rhodamine labeled tubulin was examined and found to be incorporated into microtubule assembling through our solution acceleration method (Table 1). From statistic results as shown in Table 1, protein delivery efficiency in cellular level is about 67%.

The function of delivered proteins was also tested by kinetic experiments in fish keratocytes. Fish keratocytes delivered rodamine labeled actin and tubulin were observed under time-lapsed fluorescence microscope. Cell migration and the incorporation of exogenous proteins were recorded. In Figure 4C, although freely formed rhodamine actin affected the observation of actin filament, stress fibers still can be observed during cell migration (*arrows*). Condensed rhodamine signals in membrane ruffling also indicate the actin localization (*arrow head*). Rhodamine labeled tubulin showed filamentous structure and dynamic activities during cell migration (Figure 4D). It was demonstrated that delivered exogenous protein can incorporated and assembled with actin and tubulin, and participated to the cytoskeletal function in cell migration.

Hence, it was successfully proved that the method of the invention can deliver larger proteins such as actin (43 KDa) or tubulin (55 KDa), and the biological function may still be maintained.

### EXAMPLE 4: Delivery and replication of bacteria

Pathogenic bacteria invade cells through several mechanisms. Listeria and Shigella has been reported the invasive pathway and molecule interaction with cell membrane proteins and cytoskeleton for invasion and intracellular transport (Gouin et al., 2005, Curr. Opin. Microbiol., 8: 35-45). Different invasive mechanisms involve different molecules on cell membrane, however, initiation of internalization causes bacteria to be rounded by vacuole. It is essential to escape from vacuole to avoid it (Higley and Way, 1997, Curr. Opin. Cell Biol., 9: 62-69). The method of the invention provides the possibility to dissect whether the internalization and vacuole formation is essential for bacteria invasion and survival (or not). First, *E. coli,* strain BL21-DE was transformed with the bacteria expression GFP vector. 2ml O.D. 0.2 (OD=600) EGFP *E.coli* was harvested in LB broth, and concentrated to 20 ul by centrifugation. 6 ul was used for each shot or add into cell culture medium as the control. Gentamycin was supplement with culture medium at least one hour to inhibit bacteria activities after PBS wash twice. For further incubation, gentamycin was always supplemented with cell culture medium. After 1 hr incubation with gentamycin, Hep G2 cells were washed with complete medium and observed under fluorescence microscope. EGFP *E.coli* survived in Hep G2 cells was recorded under the microscope (Fig. 5A).

To further demonstrate the survival and bio-function in delivered EGFP *E.coli.,* replication was used as the activity assay for delivered EGFP *E.coli.* Cells were lysed and pellets subject to colony formation assay were collected in different time point as indicate in Fig. 5B (*closed square*). Co-culture EGFP *E.coli* with Hep G2 cells supplemented with gentamycin was collected in different time point for colony formation assay as the negative control (Fig. 5B, *open circle).* EGFP *E.coli* was used in equal amount under solution accelerating methods in control experiments. From the results, control experiments indicated that bacteria activities were successfully inhibited by gentamycin during extended culture time. In Fig. 5B *(closed square),* the delivered EGFP *E.coli* showed increasing colony number following further incubation, indicating that the EGFP *E.coli* can still replicate after the delivery method herein. To sum up, the survival and biological function of delivered bacteria seemed to have been maintained even after the method of the present invention.

### EXAMPLE 5: Delivery of the targets can be achieved via both direct and indirect ways

Previous studies have demonstrated that accelerated helium generated shock wave with the solution (ex. culture medium) into target cells. Shock wave was found to affect cell permeability and was used as the delivery tool (Delius and Adams, 1999, Cancer Research, 59: 5227-5232; Kodama et al., 2002, Biochimica et Biophysica Acta, 1542: 186-194; Lauer et al., 1997, Gene Therapy, 4: 710-715).

Here the effect of shock wave in the method of the invention was determined. H₂O was used to replace fluorescence probes to generate the shock wave, using the same apparatus as previously described. Fluorescent probes were added into culture medium with the same concentration as previous experiments. Results by using CHO cells as the cell model was shown in Figure 6 and Table 2. After H₂O delivery, cells were washed immediately ("H₂O" in Figure 6 and Table 2) or washed after 5 min incubation in room temperature. Results were compared with using fluorescence probes as the delivery molecules ("probes" in Figure 6 and Table 2) and the control without H₂O delivery ("incubate", Table 2). Fluorescent probes added in the culture medium before H₂O delivery did not show significant signals ("incubate", Table 2). Statistical results were calculated from recorded images and more than 1000 cells were counted from 10-15 images.

It was found that Hoechst 33258 showed a high increase by H₂O delivery with further incubation for 5 min compare with wash immediately (Figure 6A, Table 2), the efficiency of Hoechst 33258 positive cells with 5 min incubation showed equilibrium as direct bombardment (Table 2). Lucifer yellow showed the similar results as Hoechst 33258 (Figure 6B, Table 2).

These results demonstrate that the accelerating H₂O generated shock wave delivered to cells, can be used to deliver soluble molecules. To determine whether such delivery by shock wave is size dependent or not, different sizes of fluorescence labeled dextrans were used. In 3 KDa and 40 KDa dextrans, H₂O delivery with immediate wash reduced the probe delivery efficiency in consequences of direct bombardment (Figure 6C-D). However, 5 min further incubation rescued the delivery efficiency (Table 2).

Together, it was found that the delivery by H₂O bombardment is size dependent. Similar results were also found in Hep G2 cells. In conclusion, molecules can be delivered by using the novel solution accelerating methods herein, which involve direct and indirect mechanisms.

**Table 2. Molecules delivery with direct and indirect force**

| | Probe direct bombardment | H₂O | H₂O and further incubate 5min | Incubate |
|---|---|---|---|---|
| Hoechst 33258 (0.5mg/ml) | 51.23±19.68 | 15.53±8.04 | 49.33±21.97 | 0 |
| Lucifer Yellow (2.5mg/ml) | 36.7±10.74 | 19.66±6.45 | 37.94±13.45 | 0 |
| Dextran ( (10mg/ml)MW:3K) | 25.19±8.2 | 12.23±5.54 | 34.21±8.75 | 0 |
| Dextran ( (10mg/ml)MW:40K) | 22.23±3.44 | 10.02±5.47 | 22.43±8.01 | 0 |
| Dextran (MW:70K)(10mg/ml) | 26.92±19.64 | 4.01±1.35 | 16.29±5.54 | 0 |
| Dextran ( (10mg/ml)MW:500K) | 40.84±17.89 | 1.498±1.382 | 1.66±1.53 | 0 |

| | | | | |
|---|---|---|---|---|
| * Concentrations of the solutions used for delivery are shown; delivery volume = 6 ml. # Mean +/- STDEV were shown. | | | | |

One skilled in the art will readily appreciate that the present invention is well adapted to carry out the objects and obtain the ends and advantages mentioned, as well as those inherent therein. The cell lines, animals, and processes and methods for producing them are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the essence of the invention.

All patents and publications mentioned in the specification are indicative of the levels of those of ordinary skill in the art to which the invention pertains.

The invention illustratively described herein suitably may be practiced in the absence of any element or elements, limitation or limitations, which are not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

## Claims

1. A method for delivering a target into one or more cells, the method comprising:
(a) optionally, mixing the target with a liquid to form a mixture; and
(b) delivering the target into the cells by high pressure force or using a fluid at high pressure.

2. A method for indirectly delivering a target into one or more cells, the method comprising:
(a) optionally, mixing the target and the cells; and
(b) changing the condition of a (eg. outer) membrane of the cells by high pressure force or using a fluid at high pressure.

3. A method according to claim 1 or 2, which is non-particle delivery method or does not involve particles for delivery of the target.

4. A method according to any one of claims 1 to 3, wherein the target is a chemical, fluorescent compound, molecule exhibiting bio-activity, micromolecule, macromolecule or microorganism.

5. A method according to any preceding claim, wherein the target is dye, fluorescent dye, polysaccharide, pharmaceutical compound, fluorescent compound, enzyme, drug, vaccine, antigen, antibody, protein, polypeptide, RNA, DNA, promoter, enhancer, siRNA, morpholino, other regulatory sequence, bacterium or yeast.

6. A method according to claim 4, wherein the microorganism is transformed bacteria or yeast.

7. A method according to any preceding claims, wherein the liquid (or solution) is water, phosphate saline buffer or other media.

8. A method according to any preceding claim, wherein the high pressure force is produced by a gene gun or by a high pressure gas (eg. helium) or liquid (eg. water).

9. A cell useful or used in a method of any preceding claim, which can facilitate the absorption of exogenous target into the cell or has increased membrane permeability, based on the condition of the membrane of the cells, or as a result of, high pressure force.

10. A method according to any one of claims 1 to 8, or cell according to claim 9, wherein the cell is an animal, insect, plant, fungus, bacterium, rickettsia or *Chlamydia* cell.
